# EUROPÄISCHE PATENTSCHRIFT

(11) **EP 1 292 265 B1**
(45) Veröffentlichungstag und Bekanntmachung des Hinweises auf die Patenterteilung: **21.03.2007**
(21) Anmeldenummer: 01945271.3
(22) Anmeldetag: 09.06.2001
(51) Int. Cl.: A61K 8/67, A61Q 5/12

(54) **Verwendung von Vitamin B6-Derivaten zur Haarstrukturverbesserung**
Use of vitamin B6 derivatives for improving hair structure
Utilisation des dérivés de vitamine B6 pour améliorer la structure des cheveux

(30) Priorität: 20.06.2000 DE 10030313; 26.04.2001 DE 10120306
(43) Veröffentlichungstag der Anmeldung: 19.03.2003
(73) Patentinhaber: Henkel Kommanditgesellschaft auf Aktien, 40589 Düsseldorf (DE)
(72) Erfinder: KLEEN, Astrid, 40699 Erkrath (DE); NAUMANN, Frank, 40219 Düsseldorf (DE); HÖFFKES, Horst, 40595 Düsseldorf (DE); BRABÄNDER, Oliver, 46049 Oberhausen (DE)
(86) Internationale Anmeldenummer: PCT/EP2001/006557
(87) Internationale Veröffentlichungsnummer: WO 2001/097760

(56) Entgegenhaltungen:
- WO-A-98/51265
- DE-A- 19 613 567
- US-A- 4 201 235
- US-A- 5 681 554

## Beschreibung

Die Erfindung betrifft die Verwendung von Verbindungen der Gruppe Vitamin B6 und deren Derivaten zur Verbesserung der Struktur und Festigkeit des Haarkeratins und der Waschechtheit von Haarfärbungen in Zubereitungen zur topischen Applikation.

Durch regelmäßige Behandlung mit alkalischen, stark reduzierenden oder oxidierenden Chemikalien, z. B. beim Dauerwellen, Färben oder Bleichen der Haare erleidet das Haarkeratin eine Schädigung der Struktur, die sich in einem Gewichtsverlust, einer Senkung des Keratin-Schmelzpunktes und in einer zunehmenden Brüchigkeit, erschwerter Kämmbarkeit der Haare und einer Verschlechterung von Sitz und Fülle der Frisur bemerkbar macht. Ein strukturgeschädigtes Haar weist darüber hinaus oft ein stumpfes und glanzloses Aussehen auf.

Um diesem Übelstand zu begegnen, hat man haarkosmetischen Präparaten schon strukturverbessernde Wirkstoffe, z. B. Formaldehyd und Formaldehyd abspaltende Verbindungen, S-Acetylsuccinanhydrid, Ammoniumvinylphosphonat, Ammoniumphosphat, Borsäure, Oxazolidine, reduzierend wirkende Zucker, Tocopherole oder sogenannte On-Säuren (z. B. Gluconsäure) zugesetzt. Obwohl diese Stoffe eine gewisse Wirksamkeit zeigen, ist besonders bei stark geschädigtem Haar die Wirkung noch unbefriedigend. Daher bestand weiterhin die Aufgabe, haarstrukturverbessemde Zusätze aufzufinden, die sich zur Nachbehandlung des Haars nach Dauerwell- oder Färbeverfahren eignen.

Pyridoxin (Pyridoxol) und andere Verbindungen, die zur Vitamin B6-Gruppe gehören, sind in Haartonika zur Verringerung des Nachfettens und zur Stimulierung des Haarwuchses schon vorgeschlagen worden.

In EP 0678293 A2 werden topische Zusammensetzungen mit einem Gehalt von Pyridoxin-tripropionat zur Behandlung des Haars und der Haut vorgeschlagen. In EP 001079 A1 sind kosmetische Zusammensetzungen mit antiseborrhoischer Wirkung beschrieben, die Pyridoxin-tripalmitat als Wirkstoff enthalten.

Gegenstand der vorliegenden Erfindung ist die Verwendung von Vitamin B6-Derivaten der Formel I, 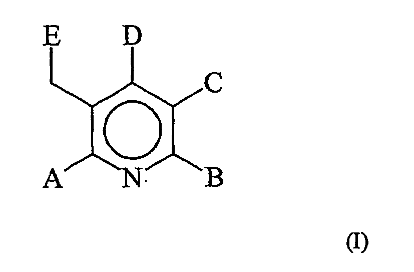 worin
- A und B stehen unabhängig voneinander für Wasserstoff, Halogen, eine C₁-C₄-Alkylgruppe, eine C₃-C₆-Cycloalkylgruppe, eine C₁-C₄-Monohydroxyalkylgruppe, eine C₂-C₄-Oligohydroxyalkylgruppe, eine C₁-C₄-Aminoalkylgruppe, eine Gruppe - OR oder eine Gruppe -NR¹R², in der R¹ und R² unabhängig voneinander für Wasserstoff, eine C₁-C₄-Alkylgruppe oder eine C₁-C₄-Monohydroxyalkylgruppe stehen, oder R¹ und R² zusammen mit dem Stickstoffatom einen gesättigten Ring bilden,
- C steht für eine Gruppe -OR, -NR¹R², -OP(O)(OR³)₂, eine C₁-C₄-Monohydroxyalkylgruppe, eine C₂-C₄-Oligohydroxyalkylgruppe oder eine C₁-C₄-Alkylgruppe,
- D steht für eine Gruppe -OR, eine Carboxygruppe, eine C₁-C₂₂-Alkoxycarbonylgruppe, einen Formylrest, eine Gruppe -CH₂OR oder eine Gruppe -CH₂-NR₂.
- E steht für eine Gruppe -OR, -OP(O)(OR³)₂, eine C₁-C₄-Monohydroxyalkylgruppe oder eine C₂-C₄-Oligohydroxyalkylgruppe,
   wobei
   - R jeweils steht für Wasserstoff, eine C₁-C₄-Alkylgruppe, eine C₁-C₂₂-Acylgruppe, eine Hydroxy-C₂-C₂₂-acylgruppe, eine C₂-C₁₀-Carboxy- acylgruppe, eine C₃-C₁₀-Oligocarboxyacylgruppe, eine Oligocarboxy-monohydroxy-C₃-C₁₀-acylgruppe, eine Oligocarboxy-oligohydroxy-C₃-C₁₀-acylgruppe, eine C₃-C₈-Cycloalkylgruppe, eine C₁-C₄-Monohydroxyalkylgruppe, eine C₂-C₄-Oligohydroxyalkylgruppe, eine Arylgruppe, welche eine Hydroxy-, Nitro- oder Aminogruppe enthalten kann, einen heteroaromatischen Rest oder eine Gruppe -CH₂CH₂NR¹R², in der R¹ und R² wie oben definiert sind,
   - R³ jeweils steht für Wasserstoff oder eine C₁-C₅-Alkylgruppe,
   oder eines der entsprechenden physiologisch verträglichen Salze, zur Verbesserung der Struktrur und Festigkeit des Haarkeratins und der Waschbeständigkeit von Haarfärbungen durch topische Applikation diese enthaltender Zubereitungen.

Bevorzugt sind Verbindungen der Formel I, in denen eine der beiden Gruppen A oder B für Wasserstoff steht.

Bevorzugt werden Verbindungen der Formel I, in denen eine der beiden Gruppen A oder B Wasserstoff und die andere Gruppe eine C₁-C₄-Alkylgruppe ist.

Ebenfalls bevorzugt sind Verbindungen der Formel I, bei denen C für eine Hydroxygruppe, eine C₁-C₄-Monohydroxyalkylgruppe oder eine C₂-C₄-Oligohydroxyalkylgruppe steht.

Die Verbindungen der Formel I werden erfindungsgemäß bevorzugt, in denen D für eine Hydroxymethylgruppe, eine Hydroxygruppe, eine Carboxygruppe, eine Gruppe -CH₂-NR₂, oder einen Formylrest steht.

Ferner sind Verbindungen der Formel I bevorzugt, in denen E eine Hydroxygruppe oder eine Gruppe -OP(O)(OH)₂ ist.

Besonders bevorzugte Verbindungen nach Formel I sind Pyridoxin (A = H, B = CH₃, C = OH, D = CH₂OH, E = OH), Pyridoxal (A = H, B = CH₃, C = OH, D = CHO, E = OH), Pyridoxal-5'-phosphat (A = H, B = CH₃, C = OH, D = CHO, E = OP(O)(OH)₂) und Pyridoxamin (A = H, B = CH₃, C = OH, D = CH₂NH₂, E = OH).

Beispiele für C₁-C₄-Alkylgruppen in den erfindungsgemäßen Verbindungen sind Methyl, Ethyl, n-Propyl, Isopropyl, n-Butyl und tert-Butyl. Bevorzugte Alkylgruppen sind Methyl und Ethyl, Methyl ist eine besonders bevorzugte Alkylgruppe. Bevorzugte C₃-C₆-Cycloalkylgruppen sind Cyclopropyl, Cyclobutyl, Cyclopentyl, Cyclohexyl. Cyclohexyl und Cyclopentyl sind besonders bevorzugte Gruppen. Bevorzugte C₁-C₄-Monohydroxyalkylgruppen sind die Gruppen Hydroxymethyl, 2-Hydroxyethyl, 3-Hydroxypropyl oder 4-Hydroxybutyl; Hydroxymethyl und 2-Hydroxyethyl sind besonders bevorzugte Monohydroxyalkylgruppen. Eine bevorzugte C₂-C₄-Oligohydroxyalkylgruppe ist die 1,2-Dihydroxyethylgruppe. Bevorzugte C₁-C₂₂-Acylgruppen sind beispielsweise Acetyl, Propionyl, Butyryl, Valeryl, Capryl, Lauryl, Myristyl, Palmityl, Stearyl, Linolyl, Behenyl. Beispiele für eine Hydroxy-C₂-C₂₂-acylgruppe sind Salicylsäure oder Milchsäure. Bevorzugte C₂-C₁₀-Carboxyacylgruppen leiten sich beispielsweise ab von der Malonsäure, Bernsteinsäure oder Adipinsäure. Ein Beispiel für eine bevorzugte C₃-C₁₀-Oligocarboxyacylgruppe ist die Glycerinsäure. Eine bevorzugte Oligocarboxy-monohydroxy-C₃-C₁₀-acylgruppe leitet sich z.B. von der Zitronensäure oder Äpfelsäure ab. Bevorzugte Oligocarboxy-oligohydroxy-C₃-C₁₀-acylgruppen sind z.B. abgeleitet aus Weinsäure. Als Halogensubstituenten eignen sich erfindungsgemäß bevorzugt Fluor, Chlor, Brom und Iod, besonders bevorzugt sind Chlor und Brom. Unter physiologisch verträglichen Salzen werden Salze anorganischer oder organischer Säuren, z. B. Hydrochloride, Sulfate oder Hydrobromide, verstanden. Die weiteren verwendeten Begriffe leiten sich erfindungsgemäß von den hier gegebenen Definitionen ab.

Die Ester-Derivate der Verbindungen gemäß Formel (I) weisen auch physiologische und die Haarstruktur verbessernde Eigenschaften auf. Dies gilt insbesondere für die Ester des Pyridoxins, die durch Hydrolyse in das Pyridoxin übergehen können. Darüber hinaus erlangen die Esterderivate eine verbesserte Lipidlöslichkeit verglichen mit den nicht veresterten Derivaten. Weitere Beispiele für Carbonsäure-Esterderivate des Pyridoxins leiten sich ab von den Carbonsäuren wie Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propanticarbonsäure, sowie von den Dicarbonsäuren ausgewählt aus der Gruppe, die gebildet wird durch Verbindungen der allgemeinen Formel (II), 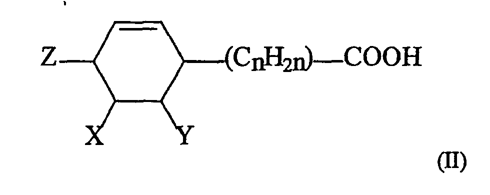 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (II), die zusätzlich noch 1 bis 3 Methyl- oder Ethylsubstituenten am Cyclohexenring tragen sowie Dicarbonsäuren, die aus den Dicarbonsäuren gemäß Formel (II) formal durch Anlagerung eines Moleküls Wasser an die Doppelbindung im Cyclohexenring entstehen.

Die strukturverbessernde Wirkung der Vitamin B6-Derivate auf das Haarkeratin kann man durch hochdruck-differenzkalorimetrische Messungen (HP-DSC) des Keratin-Schmelzverhaltens quanititativ bestimmen (vgl. Beispielteil). Die Verbesserung der Waschbeständigkeit der Haare kann man durch farbmetrische Messung des Farbabstandes zwischen dem gefärbten, ungewaschenen und dem gefärbten, mehrfach gewaschenen Haar quantitativ bestimmen. Diese Effekte sind besonders auffallend, wenn man ein stark strapaziertes Haar mit einer Zusammensetzung behandelt, die Vitamin B6-Derivate enthält. Solch stark strapaziertes Haar entsteht insbesondere durch oxidierende oder reduzierende Behandlungsverfahren, also z. B. beim Färben der Haare mit Oxidationsfärbemitteln, beim Bleichen der Haare mit Oxidationsmitteln oder beim Verformen, z. B. beim Dauerwellen oder Glätten der Haare mit starken Keratinreduktionsmitteln, z. B. mit Thioglycolat-Salzen oder mit Sulfiten.

Ein weiterer Gegenstand der Erfindung ist daher ein Verfahren zur oxidierenden oder reduzierenden Behandlung der Haare, bei dem man das Haar unmittelbar im Anschluß an die oxidierende oder reduzierende Behandlungsstufe mit einer Zusammensetzung nachbehandelt, die ein Vitamin B6-Derivat der Formel I in einer Menge von 0,05-2 Gew.-% enthält. Diese Zusammensetzung zur Nachbehandlung kann dabei ein Shampoo, eine Spülung, eine Pflegelotion, eine Festigerlotion, ein Schaum, ein Haarwasser oder ein Spray sein, d. h. es kann sich um ein "rinse-off" oder um ein "leave on"-Produkt handeln.

Wenn die Zusammensetzung nach dem Haarefärben angewandt wird, kann es sich zum Beispiel um eine wässrige Pflegelotion handeln. Wenn die Nachbehandlung nach der reduzierenden Stufe einer Dauerwellbehandlung erfolgen soll, kann das Vitamin B6-Derivat der Dauerwellfixierung zugesetzt werden. Die Nachbehandlung kann aber auch im Anschluß an die Dauerwellfixierung, die ja selbst eine oxidierende Haarbehandlung darstellt, erfolgen. In diesem Falle wird das Vitamin B6-Derivat die Form einer Pflegelotion angewandt. Bevorzugt wird die Zusammensetzung mit dem Vitamin B6-Derivat als Pflegelotion nach der oxidativen Haarfärbung oder zur Dauerwellfixierung verwendet. Neben dem Vitamin B6-Derivat kann diese Zubereitung alle für die Formulierung von Haarpflegemitteln üblichen und für das Haar oder die Kopfhaut zuträglichen Komponenten enthalten.

Die Zubereitungen zur Durchführung der oxidativen Fixierung im Dauerwellverfahren enthalten als Oxidationsmittel z. B. Wasserstoffperoxid und die zur Stabilisierung wässrigen Wasserstoffperoxidzubereitungen üblichen Stabilisatoren. Der pH-Wert solcher wässriger H₂O₂-Zubereitungen, die etwa 0,5 bis 3 Gew.-% H₂O₂ enthalten, liegt bevorzugt bei 2 bis 4; er wird mit anorganischen Säuren, bevorzugt mit Phosphorsäure, eingestellt. Das bevorzugte Oxidationsmittel ist jedoch Natrium- oder Kaliumbromat. Diese Bromate werden in Konzentration von 1 bis 10 Gew.-% eingesetzt und der pH-Wert der Zubereitungen auf 4 bis 7 eingestellt.

Außer den Oxidationsmitteln können diese Dauerwellfixiermittel weitere Komponenten, z. B. oberflächenaktive Stoffe, quartäre Ammoniumsalze, kationische Polymere, wasserlösliche Proteine- oder Proteinderivate, Duftstoffe, Trübungsmittel u.s.w enthalten. Eine Pflegelotion, die nach der Dauerwellfixierung angewendet wird, kann z. B. festigende, konditionierende, pflegende oder antistatische Komponenten, Erdalkali- oder Aluminiumsalz und andere Komponenten enthalten.

Von besonderer Bedeutung ist das erfindungsgemäße Verfahren zur Strukturverbesserung des Haarkeratins im Zusammenhang mit der oxidativen Färbung und/oder Bleichung der Haare. Besonders bevorzugt ist daher eine Haarpflegelotion, insbesondere zur Anwendung nach dem Färben oder Bleichen der Haare, die
- 1-10 Gew.-% emulgierter Öl-, Fett- oder Wachskomponenten
- 0,1 - 5 Gew.-% kationischer Tenside
- 0,2-2 Gew.-% eines Vitamin B6-Derivats der Formel I
in einem wässrigen Träger enthält.

Als Öl- oder Fettkomponenten eignen sich Paraffine, Silikonöle, pflanzliche Öle und tierische Fette (Triglyceride), Fettsäurefettalkoholester (Wachsester), Fettsäureester kurzkettiger Alkohole, Dicarbonsäure-Fettalkoholester, lineare und verzweigtkettige Alkohole und Diole mit 10-20 C-Atomen, Fettsäuremonoglyceride und andere Öle, Fette oder Wachse.

Als kationische Tenside eignen sich Verbindungen mit einer freien oder substituierten Aminogruppe oder quartären Ammoniumgruppe, die am Stickstoffatom eine oder zwei längerkettige Alkyl- oder Acylaminoalkyl- oder Acyloxyalkylgruppen und bis zu drei kurzkettigen Alkylgruppen und ggf. eine Benzylgruppe enthalten.

Beispiele für geeignete quartäre Ammoniumtenside sind z. B.
- Cetyltrimethylammonium-chlorid
- Lauryl-dimethyl-benzyl-ammonium-bromid
- Stearyl-trimethylammonium-chlorid
- Di-stearoyloxyethyl-dimethylammonium-methoxysulfat
- Kokosacylaminopropyl-trimethylammonium-chlorid

Andere kationische Tenside sind z. B. längerkettige primäre, sekundäre oder tertiäre Amine in Form ihrer Salze, z. B. der Hydrochloride oder Sulfate.

Zusätzlich zu den genannten Komponenten können erfindungsgemäße Haarpflegelotionen z.B. nichtionogene Tenside und Emulgatoren (für die Öl- und Fettkomponente), wasserlösliche Proteine, Proteinabbauprodukte oder Proteinderivate, Aminosäuren (z. B. Serin), wasserlösliche kationische Polymere, festigende, filmbildende Polymere wie z. B. Polyvinylpyrrolidon, Glucose und andere strukturierende oder die Kopfhaut pflegende Wirkstoffe wie z. B. Panthenol, Tocopherol, Allantoin, Pflanzenextrakte wie z. B. Birken- oder Kamillen-Extrakt, Antischuppenwirkstoffe, Vitamine, z. B. Vitamin B (Thiamin), B2 (Riboflavin), B3 (Nicotinsäure, Nicotinsäureamid), B7 (Biotin), B12 (Cyanocobalamin), C (Ascorbinsäure), E (Tocopherolacetat), Duftstoffe und Lichtschutzmittel enthalten.

Lichtschutzmittel haben eine besondere Bedeutung für den langzeitigen Schutz des Haarkeratins vor den schädlichen Einflüssen der Sonne. In einer bevorzugten Ausführung enthält die erfindungsgemäße Haarpflegelotion daher auch ein Lichtschutzmittel, bevorzugt in der Form einer Ultraviolettstrahlen absorbierenden Substanz. Solche Substanzen sind in großer Zahl im Handel. Dabei unterscheidet man wasserlösliche und lipidlösliche Lichtschutzmittel. In einer bevorzugten Ausführung enthält die erfindungsgemäße Pflegelotion eine Kombination aus wasserlöslichen und lipidlöslichen Lichtschutzmitteln.

Geeignete wasserlösliche Lichtschuztmittel sind z. B.:
- 2-Hydroxy-4-methoxy-benzophenon-5-sulfonsäure
- p-Methoxyzimtsäure-Diethanolaminsalz
- p-Aminobenzoesäure
- 2-Phenyl-benzimidazol-5-sulfonsäure
- Triethanolamin-Salicylat und
- Lauryl-[3-(p-Dimethylaminobenzamido)-propyl]-dimethylammonium-p-Toluolsulfonat

Geeignete lipidlösliche Lichtschutzmittel sind z. B.
- 2-Hydroxy-4-methoxy-benzophenon
- 2-Aminobenzoesäure-menthylester
- 4 Bis (2-hydroxypropyl)-aminobenzoesäure-ethylester
- 4-Aminobenzoesäure-2,3-dihydroxypropyl-ester
- 2-Ethylhexyl-2-cyano-3,3-diphenyl-acrylat
- p-Methoxyzimtsäure-2-ethylhexylester
- 4-Methylbenzyliden-campher
- 4-tert.-Butyl-4'-methoxy-dibenzoylmethan

Die Pflegelotion liegt bevorzugt in der Form einer Öl-in-Wasser Emulsion der emulgierten Öl-oder-Fettkomponenten vor, die entweder schon zum Auswaschen des überschüssigen Färbemittels aus dem Haar verwendet wird, oder die nach dem Ausspülen des überschüssigen Farbstoffs oder des überschüssigen Bleichmittels mit Wasser in einem separaten Arbeitsgang auf das Haar aufgetragen und gleichmäßig im Haar verteilt wird.

Wenn die Pflegelotion auf dem Haar verbleiben soll (leave-on-Anwendung), wird der Gehalt an emulgierten Öl- oder Fettkomponenten niedrig, bevorzugt unter 3 Gew.-% gehalten. Üblicherweise wird aber nach der Behandlung mit der Pflegelotion noch eine Spülung mit klarem Wasser vorgenommen.

Die folgenden Beispiele sollen den Gegenstand der Erfindung näher erläutern:

### Beispiele:

### 1. Nachweis der strukturgebenden Wirkung von Vitamin B6 bei topischer Applikation:

### 1.1 Prüfmethodik

### HP-DSC (High Pressure Differential Scanning Calorimetry)

Thermoanalytische Untersuchungen eignen sich besonders zur Charakterisierung von Zweiphasensystemen, zu denen die Humanhaare als Faserkeratine mit ihrem kristallinen α-Helix-Anteil und amorphen Matrix-Anteil ebenfalls gehören. Auf der einen Seite können Glasübergänge und Alterungsverhalten der amorphen Matrix untersucht werden, auf der anderen Seite liefert das Schmelzverhalten der kristallinen, helicalen Phase wichtige Erkenntnisse. Thermoanalytische Untersuchungen sind erstmals 1899 beschrieben, erste Differenzthermoanalysen (DTA) an Proteinfasern wurden Ende der fünfziger Jahre durchgeführt (F. Schwenker, J.H. Dusenbury, Text. Res. J. 1963, 30, Seite 800 ff; W. D. Felix, M.A. McDowall, H. Eyring, ibid. (1963), 33, Seite 465 ff.). In den folgenden Jahren sind unterschiedliche thermoanalytische Meßverfahren, wie DTA, HP-DTA (High Pressure, Hochdruck-DTA) und DSC (Differential Scanning Calorimetry, Dynamische Differenz-Kalorimetrie), an Keratinfasern angewendet worden um z.B. das Phänomen der Superkontraktion, α-β-Phasenübergänge der Helices oder Denaturierungsvorgänge zu untersuchen. In jüngster Zeit wird, insbesondere am Deutschen Wollforschungsinstitut in Aachen (F.J. Wortmann, H. Deutz, J. Appl. Polym. Sci. 1993, 48, Seite 137ff.), die Methode der HP-DSC zur Untersuchung von Keratinfasern genutzt, die die Probleme mit pyrolytischen Effekten, wie sie bei der konventionellen DSC auftreten, und Probleme mit der Datenerfassung und -interpretation, wie sie die DTA birgt, ausschließt. Dabei werden DSC-Messungen an Keratinen durchgeführt, die mit Wasser in kommerziell erhältlichen, druckfesten Meßkapseln eingeschlossen sind. Im Keratin-Wasser-System entwickelt sich beim Erhitzen oberhalb von 100°C in den verkapselten Stahltiegeln ein Wasserdampfhochdruck, woraus sich die HP-DSC Analyse ableitet. Der gravierende Unterschied der HP-DSC-Thermogramme von Humanhaaren im Vergleich zu normalen DSC-Thermogrammen ist der, daß die endothermen Peaks, die den Umwandlungspunkt und Umwandlungsenthalpie wiedergeben, hier um ca. 90 °C zu niedrigeren Temperaturen verschoben sind. Das rührt daher, daß das Wasser nach Diffusion in die Haarfaser durch Schwächung und Spaltung von Wasserstoffbrücken- und Salzbindungen die Proteinstabilität vermindert und so die "Verleimungstemperatur" der Keratine herabsetzt. Werden durch das superkontrahierende Agens wie Wasser nur Wasserstoffbrücken und Salzbrükken gelöst, so ist der thermische Effekt reversibel (Superkontraktion). Der Vorgang wird jedoch irreversibel, sobald auch kovalente Bindungen, wie z. B. Disulfidbrücken, gespalten werden. Dies tritt ein, wenn man Humanhaarfasern in druckfesten Kapseln mit Wasser auf über 150 °C erhitzt. Die irreversible Umwandlung, interpretiert als Übergang der α-helicalen Bereiche in den Proteinen in einen ungeordneten Zustand, resultiert in endothermen Peaks, wobei die Peaklage den Umwandlungs- oder auch Denaturierungspunkt und die Peakfläche die Umwandlungs- oder Denaturierungs-Enthalpie wiedergibt.

Unter Verwendung der Dynamischen Differenz-Kalorimetrie (DSC) können demnach strukturelle sowie chemische Zustände und Veränderungen in Faserkeratinen und insbesondere in Humanhaaren erfaßt werden. Unter genau definierten Versuchsbedingungen kann man bei Humanhaaren die kalorimetrisch erfaßbaren Vorgänge anhand von Thermogrammen aufzeichnen und sie bezüglich der Peaklagen, -strukturen und -flächen als Indikator für die Beeinflussung von Ordnungs-Unordnungsübergangen durch Änderungen innerer und/oder äußerer Parameter, hervorrufen z. B. durch kosmetische Behandlung der Haare, verwenden. D. h. aus den im Thermogramm von Humanhaaren aufgezeichneten endothermen Peaks lassen sich aufgrund von Peaklage (Umwandlungspunkt) und Peakfläche (Umwandlungsenthalpie) Aussagen über Festigkeit bzw. Schädigung der Humanhaarfaser treffen.

Ausführliche Untersuchungen bezüglich des Einflusses des Cystingehaltes auf die Denaturierung der α-Helices in Keratinen haben z. B. gezeigt, daß die Denaturierungs-Temperatur (Übergangstemperatur) des Keratins linear mit dem Cystingehalt ansteigt. Die erhöhte Stabilität des Matrixbereichs aufgrund des höheren Vernetzungsgrades des erhöhten Anteils an Disulfidbrücken in der Matrix führt dazu, daß die Umwandlung der in diese Matrix eingebetteten Helices erschwert wird und resultiert somit in einer Erhöhung der Denaturierungs-Temperatur. Umgekehrt kann in der Regel eine Denaturierungs-Temperatur- und vor allem Denaturierungs-Enthalpieerniedrigung bei durch Dauerwelle oder Bleichung bzw. Färbung behandelten Humanhaaren beobachtet werden (H. Deutz, Doktorarbeit, RWTH Aachen 1993).

### 1.2 Durchführung

Humanhaar (Alkinco 6633 und Alkinco 6634) wurde gezielt durch eine Dauerwellbehandlung (Marktprodukt Poly Lock extra starke Dauerwelle, 40 min. Dauerwelle, 10 min. Fixierung) geschädigt. Anschließend wurden jeweils 0,5 g Strähnen beider Haarqualitäten für 10 min mit einer 1%igen wäßrigen Lösung der in Tabelle 1 aufgelisteten Wirkstoffe behandelt und nach dem Abspülen getrocknet. Mittels thermoanalytischer Untersuchungen wurde dann auf (re)strukturierende Effekte der Wirkstoffe geprüft. Die erhaltenen Keratinschmelzpunkte sind in Tabelle 1 aufgelistet.

**Tab.1: (Re)strukturierung durch Vitamin B6 und Derivate**

| **Wirkstoff (1% in Wasser)** | **Keratinschmelzpunkt [°C] für Alkinco 6633** | **Keratinschmelzpunkt [°C] für Alkinco 6634** |
|---|---|---|
| --, dauergewellte Referenzprobe | 143,5 | 149,8 |
| Pyridoxin | 144 * | 152,1 |
| Pyridoxal | 147,7 | 152 |
| Pyridoxamin | 147,9 | 152,2 |
| Pyridoxal-5-Phosphat | 148,3 | 152,7 |

Mit Ausnahme der Schmelzpunkt-Erhöhung um 0,5 °C durch Pyridoxin* bei Alkinco 6633 sind alle Effekte statistisch hochsignifikant.

Durch Einsatz von Pyridoxin in einer Emulsionsformulierung (Anwendungsbeispiel 3.1) konnte ebenfalls ein in Abh. von der Einsatz-Konzentration steigender (re)strukturierender Effekt nachgewiesen werden. Auch hier wurden thermoanalytische Untersuchungen an Humanhaar (Alkinco 6634) durchgeführt, das zum Nachweis des gewünschten Effektes - wie oben beschrieben - gezielt vorgeschädigt wurde und anschließend 2 min. mit dem Pyridoxin in unterschiedlichen Konzentrationen enthaltenden Conditioner (Anwendungsbeispiel 3.1) nachbehandelt wurde. Die Ergebnisse sind in Tabelle 2 aufgeführt.

**Tab.2: (Re)strukturierung durch Vitamin B6 in Abh. von der Konz.**

| **Nachbehandlung Conditioner + x% Vitamin B6** | **Keratinschmelzpunkt [°C] Alkinco 6634** |
|---|---|
| --, dauergewellte Referenzprobe | 145,6 |
| 0 % | 145,9 |
| 0,2 % | 146,2 |
| 0,5 % | 146,5 |
| 1,0 % | 147,4 |

Bereits der durch den Einsatz von 0,2 % Vitamin B6 hervorgerufene Effekt ist im Vergleich zur Referenzprobe statistisch signifikant.

### 2. Nachweis der Verbesserung der Waschbeständigkeit von Haarfärbungen

Eine Verbesserung der Waschechtheit von Haarfarben, insbesondere Oxidationshaarfarben mit Rot- oder Violettreflex durch Vitamin B6 und Derivate konnte durch folgende Versuche nachgewiesen werden:

Es wurden Humanhaare (Kerling Naturweiß) verwendet. Die Haare wurden im Spitzenbereich gezielt geschädigt (2-fach blondiert und 2-fach dauergewellt), um stark strapaziertes Haar zu simulieren. Die Humanhaare wurden mit einer Rot-Braun-Nuance einer oxidativen Haarfarbe (Poly Diadem 718 - Haselnuß) nach Gebrauchsanweisung eingefärbt, gespült und anschließend mit einer Spülung (Anwendungsbeispiel 3.2), enthaltend 2 % Aktivsubstanz Vitamin B6 bzw. Derivat (Referenz ohne Wirkstoff) für 5 min. nachbehandelt. Nach Spülen und Trocknen wurden farbmetrische Messungen (Haarlängen und - spitzen gesondert) durchgeführt, anschließend 6x gewaschen und wiederum farbmetrische Messungen durchgeführt. Die ermittelten Werte für den Gesamtfarbabstand DE* (=Farbabstand Muster bzw. Referenz zu entsprechendem ungewaschenem, gefärbtem Haar) sind in Tabelle 3 aufgeführt. Je kleiner DE*, desto geringer der Farbverlust durch Shampoonierung und desto höher die Waschechtheit.

**Tab.3: Verbesserung der Waschechtheit durch Vitamin B6 und Derivate**

| **Haarprobe** | **DE* normale bewitterte Längen** | **DE* stark strapazierte Spitzen** |
|---|---|---|
| gefärbt, ungewaschen | 0 | 0 |
| gefärbt, Conditioner ohne Wirkstoff, 6x gewaschen | 3,2 | 8,7 |
| gefärbt, Conditioner mit 2 % Vitamin B6, 6x gewaschen | 1,8 | 5,8 |
| gefärbt, Conditioner mit 2 % Pyridoxamin, 6x gewaschen | 2,7 | 5,2 |

Die erhöhte Waschechtheit der Haarfarben ist - insbesondere auf den stark strapazierten Spitzen - auch mit dem bloßen Auge gut zu erkennen.

### 3. Anwendungsbeispiele (Haarpflegelotionen)

| **Rezeptur** | **3.1** | **3.2** |
|---|---|---|
| Paraffinöl | 3,0 Gew.-% | 2,5 Gew.-% |
| Cetyl-/Stearylalkohol | 3,0 Gew.-% | 2,5 Gew.-% |
| Fettalkohol (C₁₂₋₁₈)-polyglycolether (9,5 EO) | 0,2 Gew.-% | 0,2 Gew.-% |
| Palmitinsäure-Cetylester | 0,8 Gew.-% | 0,8 Gew.-% |
| Bienenwachs | 0,05 Gew.-% | - |
| Escalol^{®}HP 610 | 0,1 Gew.-% | - |
| Uvinul^{®} M 40 | 0,5 Gew.-% | - |
| Dehyquart^{®} A | 3,0 Gew.-% | 3,0 Gew.-% |
| Phenoxyethanol | 0,6 Gew.-% | 0,6 Gew.-% |
| Kamillen-Destillat | 0,6 Gew.-% | - |
| Methyl-hydroxypropyl-cellulose | 0,6 Gew.-% | 0,6 Gew.-% |
| pHB-methylester | 0,2 Gew.-% | 0,2 Gew.-% |
| pHB-propylester | 0,1 Gew.-% | 0,1 Gew.-% |
| Pyridoxin | 0 - 1,0 Gew.-% | 2,0 Gew.-% |
| Parfüm | 0,3 Gew.-% | 0,15 Gew.-% |
| Wasser | ad 100 (Gew.-%) | ad 100 (Gew.-%) |

Es wurden die folgenden Handelsprodukte verwendet:
- Escalol^{®} HP 610: Dodecyl- [3(p-Dimethylaminobenzamido)-propyl]-dimethylammonium-p-toluolsulfonat mit Propylenglycolmonostearat (QAV min 65 %, H₂O: 12-18 %)
- Uvinul^{®} M40: 2-Hydroxy-4-methoxybenzophenon
- Dehyquart^{®} A: Cetyltrimethylammonium-chlorid (25%ig in Wasser)

## Patentansprüche

1. Verwendung von Vitamin B6-Derivaten der Formel I, 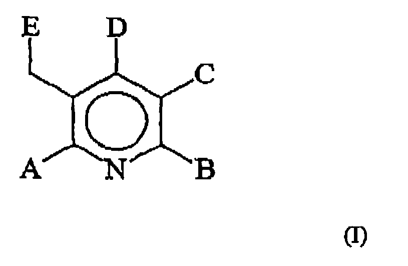 worin
- A und B stehen unabhängig voneinander für Wasserstoff, Halogen, eine C₁-C₄-Alkylgruppe, eine C₃-C₆-Cycloalkylgruppe, eine C₁-C₄-Monohydroxyalkylgruppe, eine C₂-C₄-Oligohydroxyalkylgruppe, eine C₁-C₄-Aminoalkylgruppe, eine Gruppe -OR oder eine Gruppe -NR¹R², in der R¹ und R² unabhängig voneinander für Wasserstoff, eine C₁-C₄-Alkylgruppe oder eine C₁-C₄-Monohydroxyalkylgruppe stehen, oder R¹ und R² zusammen mit dem Stickstoffatom einen gesättigten Ring bilden,
- C steht für eine Gruppe -OR, NR¹R², -OP(O)(OR³)₂, eine C₁-C₄-Monohydraxyalkylgruppe, eine C₂-C₄-Oligohydroxyalkylgruppe oder eine C₁-C₄-Alkylgruppe,
- D steht für eine Gruppe -OR, eine Carboxygruppe, eine C₁-C₂₂-Alkoxycarbonylgruppe, einen Formylrest eine Gruppe -CH₂OR oder eine Gruppe
- CH₂-NR₂,
- E steht für eine Gruppe -OR, -OP(O)(OR³)₂, eine C₁-C₄-Monohydroxyalkylgruppe oder eine C₂-C₄-Oligohydroxyalkylgruppe,
wobei
- R jeweils steht für Wasserstoff, eine C₁-C₄-Alkylgruppe, eine C₁-C₂₂-Acylgruppe, eine Hydroxy-C₂-C₂₂-acylgruppe, eine C₂-C₁₀-Carboxyacylgruppe, eine C₃-C₁₀-Oligocarboxyacylgruppe, eine Oligocarboxymonohydxoxy-C₃-C₁₀-acylgruppe, eine Oligocarboxy-oligohydroxy-C₃-C₁₀-acylgruppe, eine C₃-C₈-Cycloalkylgruppe, eine C₁-C₄-Monohydroxyalkylgruppe, eine C₂-C₄-Oligohydroxyalkylgruppe, eine Arylgruppe, welche eine Hydroxy-, Nitro- oder Aminogruppe enthalten kann, einen heteroaromatischen Rest oder eine Gruppe -CH₂CH₂NR¹R², in der R¹ und R² wie oben definiert sind,
- R³ jeweils steht für Wasserstoff oder eine C₁-C₅-Alkyllgruppe,
oder eines der entsprechenden physiologisch verträglichen Salze, zur Verbesserung der Struktrur und Festigkeit des Haarkeratins und der Waschbeständigkeit von Haarfärbungen durch topische Applikation diese enthaltender Zubereitungen.

2. Verwendung nach Anspruch 1, **dadurch gekennzeichnet, daß** eine der beiden Gruppen A oder B für Wasserstoff steht.

3. Verwendung nach einem der Ansprüche 1 oder 2, **dadurch gekennzeichnet, daß** eine der beiden Gruppen A oder B Wasserstoff und die andere Gruppe eine C₁-C₄-Alkylgruppe ist.

4. Verwendung nach einem der Ansprüche 1 bis 3, **dadurch gekennzeichnet, daß** C für eine Hydroxygruppe, eine C₁-C₄-Monohydroxyalkylgruppe oder eine C₂-C₄-Oligohydroxy-alkylgruppe steht.

5. Verwendung nach einem der Ansprüche 1 bis 4, **dadurch gekennzeichnet, daß** D für eine Hydroxymethylgruppe, eine Hydroxygruppe, eine Carboxygruppe, eine Gruppe -CH₂-NR₂ oder einen Formylrest steht.

6. Verwendung nach einem der Ansprüche 1 bis 5, **dadurch gekennzeichnet, daß** E für eine Hydroxygruppe oder eine Gruppe -OP(O)(OH)₂ steht.

7. Verwendung nach einem der Ansprüche 1 bis 6, **dadurch gekennzeichnet, daß** als Vitamin B6-Derivat eine Verbindung aus der Gruppe Pyridoxin, Pyridoxal, Pyridoxal-5'-phosphat und Pyridoxamin eingesetzt wird.

8. Verfahren zur oxidierenden oder reduzierenden Behandlung von Haaren, **dadurch gekennzeichnet, daß** man das Haar unmittelbar im Anschluß an die oxidierende oder reduzierende Behandlungsstufe mit einer Zusammensetzung nachbehandelt, die eine Verbindung der Formel I gemäß Patentanspruch 1 in einer Menge von 0,05-2 Gew.-% enthält.

9. Verfahren nach Anspruch 8, **dadurch gekennzeichnet, daß** die Zusammensetzung als Pflegelotion nach der oxidativen Haarfärbung oder zur Dauerwellfixierung verwendet wird.

## Claims

1. The use of vitamin B6 derivatives corresponding to formula (I): 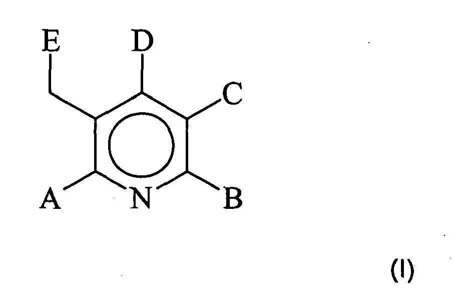 in which
- A and B independently of one another represent hydrogen, halogen, a C₁₋₄ alkyl group, a C₃₋₆ cycloalkyl group, a C₁₋₄ monohydroxyalkyl group, a C₂₋₄ oligohydroxyalkyl group, a C₁₋₄ aminoalkyl group, a group -OR or a group -NR¹R², where R¹ and R² independently of one another represent hydrogen, a C₁₋₄ alkyl group or a C₁₋₄ monohydroxyalkyl group or R¹ and R² together with the nitrogen atom form a saturated ring,
- C represents a group -OR, -NR¹R², -OP(O)(OR³)₂, a C₁₋₄ monohydroxyalkyl group, a C₂₋₄ oligohydroxyalkyl group or a C₁₋₄ alkyl group,
- D represents a group -OR, a carboxy group, a C₁₋₂₂ alkoxycarbonyl group, a formyl group, a group -CH₂OR or a group -CH₂-NR₂,
- E represents a group -OR, -OP(O)(OR³)₂, a C₁₋₄ monohydroxyalkyl group or a C₂₋₄ oligohydroxyalkyl group,
- R representing hydrogen, a C₁₋₄ alkyl group, a C₁₋₂₂ acyl group, a hydroxy-C₂₋₂₂-acyl group, a C₂₋₁₀ carboxyacyl group, a C₃₋₁₀ oligocarboxyacyl group, an oligocarboxymonohydroxy-C₃₋₁₀-acyl group, an oligocarboxyoligohydroxy-C₃₋₁₀-acyl group, a C₃₋₈ cycloalkyl group, a C₁₋₄ monohydroxyalkyl group, a C₂₋₄ oligohydroxyalkyl group, an aryl group which may contain a hydroxy, nitro or amino group, a heteroaromatic group or a group -CH₂CH₂NR¹R², where R¹ and R² are as defined above,
- R³ representing hydrogen or a C₁₋₅ alkyl group,
or one of the corresponding physiologically compatible salts, for improving the structure and strength of hair keratin and the fastness to washing of hair colors by topical application of preparations containing these derivatives/salts.

2. The use claimed in claim 1, **characterized in that** one of the two groups A and B is hydrogen.

3. The use claimed in claim 1 or 2, **characterized in that** one of the two groups A and B is hydrogen and the other is a C₁₋₄ alkyl group.

4. The use claimed in any of claims 1 to 3, **characterized in that** C is a hydroxy group, a C₁₋₄ monohydroxyalkyl group or a C₂₋₄ oligohydroxyalkyl group.

5. The use claimed in any of claims 1 to 4, **characterized in that** D is a hydroxymethyl group, a hydroxy group, a carboxy group, a group -CH₂-NR₂ or or a formyl group.

6. The use claimed in any of claims 1 to 5, **characterized in that** E is a hydroxy group or a group -OP(O)(OH)₂.

7. The use claimed in any of claims 1 to 6, **characterized in that** a compound from the group consisting of pyridoxine, pyridoxal, pyridoxal-5'-phosphate and pyridoxamine is used as the vitamin B6 derivative.

8. A process for the oxidizing or reducing treatment of hair, **characterized in that**, immediately after the oxidizing or reducing treatment step, the hair is aftertreated with a composition containing a compound corresponding to formula I in claim 1 in a quantity of 0.05 to 2% by weight.

9. A process as claimed in claim 8, **characterized in that** the composition is used as a hair care lotion after oxidative coloring or for permanent wave fixing.

## Revendications

1. Utilisation de dérivés de la vitamine B6 de formule I, 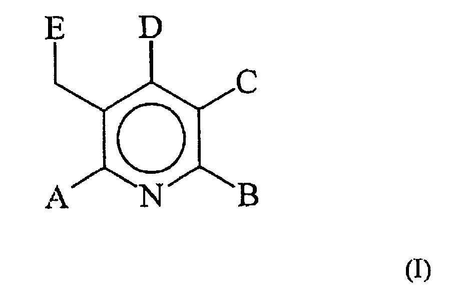 dans laquelle
- A et B représentent, indépendamment l'un de l'autre, hydrogène, halogène, un groupe C₁-C₄-alkyle, un groupe C₃-C₆-cycloalkyle, un groupe C₁-C₄-monohydroxyalkyle, un groupe C₂-C₄-oligohydroxyalkyle, un groupe C₁-C₄-aminoalkyle, un groupe -OR ou un groupe -NR¹R², où R¹ et R² représentent, indépendamment l'un de l'autre, hydrogène, un groupe C₁-C₄-alkyle ou un groupe C₁-C₄-monohydroxyalkyle, ou R¹ et R² forment ensemble avec l'atome d'azote un cycle saturé,
- C représente un groupe -OR, -NR¹R², -OP(O)(OR³)₂, un groupe C₁-C₄-monohydroxyalkyle, un groupe C₂-C₄-oligohydroxyalkyle ou un groupe C₁-C₄-alkyle,
- D représente un groupe -OR, un groupe carboxy, un groupe C₁-C₂₂-alcoxycarbonyle, un radical formyle, un groupe -CH₂OR ou un groupe -CH₂-NR₂,
- E représente un groupe -OR, -OP(O)(OR³)₂, un groupe C₁-C₄-monohydroxyalkyle ou un groupe C₂-C₄-oligohydroxyalkyle,
où:
- R représente à chaque fois hydrogène, un groupe C₁-C₄-alkyle, un groupe C₁-C₂₂-acyle, un groupe hydroxy-C₂-C₂₂-acyle, un groupe C₂-C₁₀-carboxyacyle, un groupe C₃-C₁₀-oligocarboxyacyle, un groupe oligocarboxymonohydroxy-C₃-C₁₀-acyle, un groupe oligocarboxyoligohydroxy-C₃-C₁₀-acyle, un groupe C₃-C₈-cycloalkyle, un groupe C₁-C₄-monohydroxyalkyle, un groupe C₂-C₄-oligohydroxyalkyle, un groupe aryle, qui peut contenir un groupe hydroxy, nitro ou amino, un radical hétéroaromatique ou un groupe -CH₂CH₂NR¹R², dans lequel R¹ et R² sont tels que définis ci-dessus,
- R³ représente à chaque fois hydrogène ou un groupe C₁-C₅-alkyle,
ou un sel physiologiquement acceptable correspondant, pour l'amélioration de la structure et de la résistance de la kératine des cheveux et la résistance au lavage de teintures pour cheveux par application topique de compositions qui les contiennent.

2. Utilisation selon la revendication 1, **caractérisée en ce qu'**un des deux groupes A ou B représente hydrogène.

3. Utilisation selon l'une quelconque des revendications 1 ou 2, **caractérisée en ce qu'**un des deux groupes A ou B représente hydrogène et l'autre groupe est un groupe C₁-C₄-alkyle.

4. Utilisation selon l'une quelconque des revendications 1 à 3, **caractérisée en ce que** C représente un groupe hydroxy, un groupe C₁-C₄-monohydroxyalkyle ou un groupe C₂-C₄-oligohydroxyalkyle.

5. Utilisation selon l'une quelconque des revendications 1 à 4, **caractérisée en ce que** D représente un groupe hydroxyméthyle, un groupe hydroxy, un groupe carboxy, un groupe -CH₂-NR₂ ou un radical formyle.

6. Utilisation selon l'une quelconque des revendications 1 à 5, **caractérisée en ce que** E représente un groupe hydroxy ou un groupe -OP(O)(OH)₂.

7. Utilisation selon l'une quelconque des revendications 1 à 6, **caractérisée en ce qu'**on utilise comme dérivé de la vitamine B6 un composé du groupe formé par la pyridoxine, le pyridoxal, le pyridoxal-5'-phosphate et la pyridoxamine.

8. Procédé pour le traitement par oxydation ou réduction de cheveux, **caractérisé en ce qu'**on post-traite les cheveux directement après l'étape de traitement par oxydation ou réduction avec une composition qui contient un composé de formule I selon la revendication 1 en une quantité de 0,05-2% en poids.

9. Procédé selon la revendication 8, **caractérisé en ce que** la composition est utilisée comme lotion de soin après la teinture de cheveux par oxydation ou pour la fixation d'une permanente.
